(19)

Europäisches
Patentamt

European
Patent Office

Office européen
des brevets

(11) **EP 4 108 231 A1**

(12) **EUROPEAN PATENT APPLICATION**

(43) Date of publication:
**28.12.2022 Bulletin 2022/52**

(21) Application number: **21181716.8**

(22) Date of filing: **25.06.2021**

(51) International Patent Classification (IPC):
***A61K 9/00*** *(2006.01)*

(52) Cooperative Patent Classification (CPC):
**A61K 9/0051; A61K 9/0048**

| | |
|---|---|
| (84) Designated Contracting States:<br>**AL AT BE BG CH CY CZ DE DK EE ES FI FR GB<br>GR HR HU IE IS IT LI LT LU LV MC MK MT NL NO<br>PL PT RO RS SE SI SK SM TR**<br>Designated Extension States:<br>**BA ME**<br>Designated Validation States:<br>**KH MA MD TN**<br><br>(71) Applicant: **Johann-Wolfgang-Goethe-Universität<br>Frankfurt am Main<br>60323 Frankfurt am Main (DE)** | (72) Inventors:<br>• **Windbergs, Maike**<br> **61462 Königstein im Taunus (DE)**<br>• **Walther, Marcel**<br> **60433 Frankfurt am Main (DE)**<br>• **Rohde, Felix**<br> **65474 Bischofsheim (DE)**<br><br>(74) Representative: **Krauss, Jan<br>Boehmert & Boehmert<br>Anwaltspartnerschaft mbB<br>Pettenkoferstrasse 22<br>80336 München (DE)** |

(54) **IN-SITU DISSOLVING ELECTROSPUN POLYMER FIBERS FOR OCULAR DRUG DELIVERY**

(57) The present invention relates to drug-containing polymer fibers or drug-containing fiber mats for ophthalmic use that are produced by electrospinning solutions of biocompatible polymers, and respective uses thereof.

EP 4 108 231 A1

**Description**

The present invention relates to drug-containing polymer fibers or drug-containing fiber mats for ophthalmic use that are produced by electrospinning solutions of biocompatible polymers, and respective uses thereof.

**Background of the invention**

Ophthalmologic diseases, e.g. conjunctivitis, glaucoma, age-related macular degeneration, are widespread in the population. Solely, conjunctivitis annually affects approximately 6 million people in the U.S. (1). In the last years, research has focused on understanding the mechanisms of ophthalmologic diseases and developing innovative therapy approaches. Despite the high prevalence of ophthalmologic diseases and improved understanding of disease mechanisms, there is still a lack of innovative delivery systems for adequate drug release to the anterior eye and improved patient compliance (2).

The human eye is a very sensitive application site for pharmaceutical therapy with severe damage upon therapy failure (e.g. eye irritation, microbial infection, loss of sight). The ophthalmo-physiological requirements regarding pH, osmolarity, sterility and non-cytotoxicity make ophthalmic delivery of drugs highly challenging.

Current drug delivery systems for treatment of ophthalmologic diseases still mostly comprise simple dosage forms, such as liquid eye drops and different semi-solids (3). Despite their broad patient compliance, these formulations exhibit significant drawbacks. The exact volume of an eye drop is highly dependent on the application technique. Usual volumes range from 20 to 50 $\mu$L, significantly exceeding the conjunctival sac capacity of 7-10 $\mu$L, thus causing immediate discharge and nasolacrimal drainage. Consequences are shortened contact time, increased unwanted systemic drug availability, requiring the application of higher drug loads for compensation (4,5). In order to extend the contact time, viscosity enhancing excipients, such as in-situ gelling and mucoadhesive agents are added to the formulation (6-8).

Further disadvantages associated with fluid or semi-solid formulations are instability of the API (hydrolysis and oxidation), as well as microbial instabilities. Microbiological contaminations are carried over between applications, accounting for longer treatment periods. The handling of established dosage forms is highly challenging during application and the dosage reproducibility is not given (9). After application of eye drops or semi-solid formulations, the vision is often impaired due to differences in the refractive index. Especially oily eye drops and semi-solid formulations heavily impair the vision, thus affecting patient compliance. Another drawback is the limited drug loading capacity due to the low solubility of a majority of newly approved APIs in aqueous media, leading to increased application frequency or formulation compromises (e.g. oily formulations with visual impairment or coarsely dispersed systems with thermodynamic instabilities). Such systems usually require a combination of several different excipients to stabilize the resulting eye drop system, increasing the risk for adverse effects, such as allergic reactions and irritation (10).

For adequate therapy, the drug delivery system should be capable of stabilizing the drug, ensuring reproducible dosing and prolonging contact time. Ideally, the volume of the applied dosage form should not exceed the conjunctival sac capacity. Furthermore, the application of such delivery systems should be simplified compared to eye drops and semi-solid dosage forms. As example, ocular inserts, consisting of biocompatible water-insoluble polymers, have the advantage of achieving an extended and controlled drug release, ensuring adequate ocular bioavailability and reducing the application frequency. However, these systems bear disadvantages due to the highly challenging application and removal. The insertion increases the foreign body sensation, inducing a mechanical irritation of the eye, and can furthermore impair the vision by moving over the ocular surface with the risk of losing the drug delivery system (11).

Franco and De Marco (in: The Use of Poly(N-vinyl pyrrolidone) in the Delivery of Drugs: A Review. Polymers (Basel). 2020;12(5):1114. Published 2020 May 13. doi:10.3390/polym12051114) highlight the role of PVP in drug delivery, focusing on the different morphologies proposed for different polymer/active compound formulations.

Almeida H, et al. (in: Applications of poloxamers in ophthalmic pharmaceutical formulations: an overview. Expert Opin Drug Deliv. 2013 Sep;10(9):1223-37. doi: 10.1517/17425247.2013.796360. Epub 2013 May 21. PMID: 23688342) describe poloxamers as polyols with thermal gelling properties which are frequently included in ophthalmic formulations to improve the ocular bioavailability of drugs by increasing vehicle viscosity.

WO2019148291A1 discloses ocular inserts for the sustained release of steroids in the treatment ocular conditions, comprising, for example, an antibiotic or steroid, in particular dexamethasone. The article is a fiber, fiber mesh, woven fabric, non- woven fabric, pellet, cylinder, microparticle, nanoparticle, or shaped article. The formulation may contain PVP as antioxidants. The fibers are not self-dissolvable, and have no effect on the viscosity of tear fluid.

In view of the above, it is an object of the present invention to provide improved methods and materials for delivery systems for an effective prevention or therapy of ophthalmic diseases. Other objects and advantages will become apparent to the person of skill when studying the present description of the present invention.

Generally, and by way of brief description, the main aspects of the present invention can be described as follows:

In a first aspect of the present invention the above object is solved by providing a method for producing drug-containing polymer fibers for ocular use, comprising a) providing at least one drug suitable for ophthalmic/ocular use, b) adding said at least one drug to a suitable solution of at least one biocompatible polymer, wherein said polymer is capable of incorporating and stabilizing the at least one drug, is hydrophilic, is tear fluid dissolvable and increases the viscosity of the tear fluid when brought in contact with said tear fluid, and c) electrospinning of said biocompatible polymer solution of b) in order to produce said drug-containing polymer fibers.

Preferred is a method for producing drug-containing fiber mats for ophthalmic/ocular use, comprising the method according to the present invention as above, and further comprising the step of solvent evaporation and forming fiber mats, preferably consisting of dried polymer polymer fibers.

To overcome the previously mentioned disadvantages, the present inventors have developed immediately dissolving solid eye drops prepared by electrospinning. For this purpose, in a preferred embodiment, polymer polymer fibers were loaded with dexamethasone and gentamicin, intended for the treatment of bacterial conjunctivitis. This innovative system showed distinct advantages over established ophthalmic drug delivery systems with respect to dosing accuracy, drug stabilization, and combined immediate release with prolonged contact time, while ensuring easy application without inducing a mechanical irritation to the eye.

In a second aspect of the present invention the above object is solved by drug-containing polymer fibers or drug-containing fiber mats, produced according to a method according to the present invention as above, wherein preferably the biocompatible polymer is a mixture of polyvinylpyrrolidone (PVP) and poloxamer 188, and more preferably the drug is the combination of gentamicin and dexamethasone.

In a third aspect of the present invention the above object is solved by a pharmaceutical composition, comprising an effective amount of the drug-containing polymer fibers or drug-containing fiber mats according to the present invention, preferably in the form of solid eye drops or an ophthalmic dressing.

In a fourth aspect of the present invention the above object is solved by the drug-containing polymer fibers or drug-containing fiber mat according to the present invention or the pharmaceutical composition according to the present invention for use in the prevention or treatment of diseases, in particular for use in the prevention or treatment of ophthalmologic diseases, such as conjunctivitis, infectious conjunctivitis, glaucoma, and/or age-related macular degeneration, as well as the healing of cornea defects, e.g. injuries, post-surgical recovery, preferably in the form of solid eye drops or an ophthalmic dressing.

In a fifth aspect of the present invention the above object is solved by a method for preventing or treating of an ophthalmologic disease, such as conjunctivitis, infectious conjunctivitis, glaucoma, and/or age-related macular degeneration, as well as healing of cornea defects, e.g. injuries, post-surgical recovery, comprising providing to a patient in need thereof an effective amount of the pharmaceutical composition according to the present invention.

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

As mentioned above, the present inventors have developed immediately dissolving solid eye drops prepared by electrospinning. The inventive system immediately dissolves upon application and increases the viscosity of the tear film, thus prolonging the contact time of the embedded actives and thus their pharmacological effect period. The system can easily be spread across the eye ball, thus reaching the periphery and ensuring even drug distribution, while minimizing the visual impairment. The innovative system further showed distinct advantages over established ophthalmic drug delivery systems with respect to dosing accuracy, drug stabilization, and combined immediate release with prolonged contact time, while ensuring easy application without inducing a mechanical irritation to the eye.

In a first aspect of the present invention the above object is solved by drug-containing polymer fibers or drug-containing fiber mat, produced according to a method comprising a) providing at least one drug suitable for ophthalmic/ocular use, b) adding said at least one drug to a suitable solution of at least one biocompatible polymer, wherein said polymer is capable of incorporating and stabilizing the at least one drug, is hydrophilic, is tear fluid dissolvable and increases the viscosity of the tear fluid when brought in contact with said tear fluid, and c) electrospinning of said biocompatible polymer solution of b) in order to produce said drug-containing polymer fibers. For the mat, the method further comprises the step of solvent evaporation and forming fiber mats, preferably consisting of dried polymer fibers.

In a preferred embodiment, in the drug-containing polymer fibers or drug-containing fiber mats the biocompatible polymer is a mixture of PVP and poloxamer 188. Preferably, the drug is the combination of gentamicin and dexamethasone.

A rational drug delivery system design for ophthalmic applications should be capable of addressing the following challenges associated with established systems:

- Short contact time of APIs due to discharge and nasolacrimal drainage,
- Visual impairment for prolonged fluid or semi-solid formulations (e.g. oily solid or semi-solid formulations),
- Dosing inaccuracy upon patient handling,
- Chemical and microbiological formulation instabilities,
- Excessive use of excipients and resulting corneal irritation (e.g. pH, osmotic agents, viscosity enhancers, preservatives, antioxidants), and
- Application difficulties.

The short contact time between the formulation and its application site can be overcome by including viscosity enhancing or mucoadhesive agents to fluid eye drop formulations. Another approach to prolong the contact time is the development of semi-solid dosage forms, which are usually accompanied by visual impairment. Also, both systems have the distinct disadvantage of dosing inaccuracy upon patient handling. Such dosing inaccuracy can generally be overcome by using delivery systems containing a single dose of drug, which is in most cases achieved with solid dosage forms. Such solid dosage forms have the advantage of increased chemical and microbiological stability, thus waiving respective excipients. Most extended release solid ophthalmic inserts induce a foreign body sensation, which leads to irritation of the eye.

Electrospinning is a technique for the generation of drug-loaded solid polymer polymer fiber mats. These mats are capable of stabilizing the incorporated drugs and only require minimal quantities of excipients. In brief, the polymer solution transforms into polymer fibers depositing on a collector under the influence of a high voltage (18). The preferred chosen polymers, PVP and poloxamer 188, are biocompatible and FDA approved, with long-term established use in ophthalmic dosage forms. Both preferred polymers are hydrophilic, water-soluble and show good drug compatibility. PVP as well as poloxamers have extensively been studied for ophthalmic application and are well established viscosity increasing agents in pharmaceutically marketed eyedrop formulations (19-21).

By dissolving drugs in the polymer solutions prior to electrospinning, the generated polymer fibers become drug delivery systems with scalable encapsulation efficiency. The drug load can easily be adjusted by increasing the drug concentration in the polymer solution or by electrospinning a thicker fiber mat, adapting the drug load to various clinical needs. By incorporating drugs into a solid polymer fiber matrix, the physico-chemical stability is increased, reducing the risk of oxidation, hydrolysis or phase separation exhibited by fluid or semi-solid dosage forms. Furthermore, the solid system is not susceptible to microbial contamination.

These ocular drug delivery systems consisting of PVP and poloxamer 188 instantly dissolve when applied to the ocular surface, thus instantly increasing the viscosity of the tear fluid. This leads to longer maintenance of therapeutic concentrations and reduction of application frequency.

Göttel et al. (in: Electrospun nanofibers - A promising solid in-situ gelling alternative for ocular drug delivery, Eur. J. Pharm. Biopharm. 146 (2020) 125-132. https://doi.org/10.1016/j.ejpb.2019.11.012) developed an ocular drug delivery system, which is applied in dry form and forms immediately a gel after administration. The system is based on gellan gum/pullulan electrospun nanofibers. The rheological behavior of the spinning solution was investigated followed by further characterization of the in situ formed gel. Three-dimensional X-ray imaging with nanometric resolution (nano-CT) and electron scanning microscopy were used for a detailed characterization of the diameter and alignment of the fibers. A high porosity (87.5 $\pm$ 0.5%) and pore interconnectivity (99%) was found. To ensure a good fit to the eye anatomy, the prepared fibers were shaped into curved geometries. A clear prolongation of the fluorescein residence time compared to conventional eye drops was achieved with the application of the curved nanofibers in situ gelling mat. The developed in situ gelling system with adapted geometry is described as a promising alternative system for ocular drug delivery.

Grimaudo, et al. (in: Crosslinked Hyaluronan Electrospun Nanofibers for Ferulic Acid Ocular Delivery, Pharmaceutics 12 (2020). https://doi.org/10.3390/pharmaceutics12030274) disclose the design of an ophthalmic insert composed of hyaluronan (HA) nanofibers for the dual delivery of an antioxidant (ferulic acid, FA) and an antimicrobial peptide ($\varepsilon$-polylysine, $\varepsilon$-PL). Polyvinylpyrrolidone (PVP) was added to facilitate the electrospinning process. Fibers with diameters of approx. 100 nm were obtained with PVP 5%-HA 0.8% w/v and PVP 10%-HA 0.5% w/v mixtures in ethanol:water 4:6 v/v. An increase in PVP concentration to 20% w/v in both presence and absence of HA rendered fibers of approx. 1 $\mu$m. PVP 5%-HA 0.8% w/v fibers were loaded with 83.3 $\pm$ 14.0 $\mu$g FA per mg. After nanofibers crosslinking with $\varepsilon$-PL, blank and FA-loaded inserts showed a mean thickness of 270 $\pm$ 21 $\mu$m and 273 $\pm$ 41 $\mu$m, respectively. Blank and FA-loaded inserts completely released $\varepsilon$-PL within 30 min under sink conditions, whereas FA-loaded inserts released the antioxidant within 20 min. Both blank and FA-loaded inserts were challenged against *Pseudomonas aeruginosa* and Staphylococcus aureus, demonstrating their efficacy against relevant microbial species

Both electrospun formulations presented by Göttel et al. and Grimaudo et al. as above are clearly different from the present inventive solid eye drop system.

The system by Grimaudo et al. is used for extended release purposes and for longer application durations.

Disadvantageously, the polymer matrix has to be removed upon drug release completion, whereas the present system is designed for immediate release of APIs and prolonged contact time. The present system dissolves immediately and is cleared from the eye after an extended period of time, thus no removal is necessary.

During production of the contact lenses presented by Göttel et al., the spinning solution has to be heated up to 80°C in order to enable dissolution of the polymers in water, thus limiting the range of suitable thermostable drugs. The natural polysaccharides produced in microorganisms are highly variable in their constitution. Lipophilic drugs cannot be incorporated into this system, as water is the only solvent used for preparation of the electrospinning solution. The present system is comprised of well-defined synthetic polymers with emulsifying properties to enable the incorporation of thermolabile hydrophilic as well as lipophilic drugs, which can further be promoted by tailor-made adjustment of the solvent used to prepare the electrospinning solution. Göttel et al. designed an in-situ gelling contact lens system, which forms a stiff gel with concrete borders, in which the fluorescent dye (fluorescein) can be detected for a longer period of time, but fluorescein concentration in the periphery remains unclear. The drug release from the formed gel was not investigated and remains unclear. The gel persists on the ocular surface and is not cleared, likely leading to a long lasting strongly impaired vision.

Vigani B, et al. (in: Recent Advances in the Development of In Situ Gelling Drug Delivery Systems for Non-Parenteral Administration Routes. Pharmaceutics. 2020;12(9):859. Published 2020 Sep 10. doi:10.3390/pharmaceutics12090859) provide an overview of the in situ gelling drug delivery systems developed in the last 10 years for non-parenteral administration routes, such as ocular, nasal, buccal, gastrointestinal, vaginal and intravesical ones, with a special focus on formulation composition, polymer gelation mechanism and in vitro release studies.

In a second aspect of the present invention provided is a method for producing drug-containing polymer fibers or a drug-containing fiber mat, comprising a) providing at least one drug suitable for ocular use, b) adding said at least one drug to a suitable solution of at least one biocompatible polymer, wherein said polymer is capable of incorporating and stabilizing the at least one drug, is hydrophilic, is tear fluid dissolvable and increases the viscosity of the tear fluid when brought in contact with said tear fluid, and c) electrospinning of said biocompatible polymer solution of b) in order to produce said drug-containing polymer fibers. For producing the mat, the method further comprises the step of solvent evaporation and forming fiber mats on the collector, preferably consisting dried polymer fibers.

In a preferred embodiment of the method according to the invention, PVP (15% w/v) and poloxamer 188 (2.5% w/v) were dissolved in a suitable solvent, here a mixture of double distilled water/ethanol in a ratio of 4:1 (v/v) under stirring conditions at room temperature. After complete dissolution, the drugs gentamicin (1.4% w/v) and dexamethasone (0.28% w/v) were added. The polymer solution was then electrospun with a flow rate of 2.0 mL/h at 15 kV onto a rotating drum cylinder with a diameter of 10 cm at 100 revolutions per minute (RPM). After completion, the thus formed electrospun fiber mats were stored in a desiccator at ambient temperature until further use.

In general, any biocompatible polymer suitable and acceptable for ophthalmic use, such as, for example, PVP, poloxamer, and combinations thereof, can be used in the context of the present invention. The polymers to be used for this invention may include but are not be limited to natural (e.g. gelatin, alginate, chitosan, polysaccharides, cellulose esters, cellulose ethers, gellan gum, traganth, hyaluronic acid, hydroxypropyl guar, tamarind polysaccharides, collagen, pectins, lignosulfonates, dextran), as well as synthetic polymers (e.g. poly(acrylic acid), poly(vinyl alcohol), poly(vinyl amine), poly(methacrylic acids), poly(vinyl pyrrolidone), poly(ethylene glycol), poly(ethylene oxide), poly(propylene glycol), poly(ethyleneimine), poly(acryl amide), poly(styrene sulfonates), poly(acryl amide), poly(allyl amine), poly(oxazoline)), and combinations thereof. This includes block and alternating combinations of the above mentioned polymers, as well as respective derivatives and synthetic derivatives. The polymers are hydrophilic, water-soluble and show good drug compatibility, i.e. said polymer is capable of incorporating and stabilizing the at least one drug (i.e. not causing substantial drug degradation during production and storage of the fibers). PVP as well as poloxamers, have extensively been studied for ophthalmic application and are well-established viscosity increasing agents in pharmaceutically marketed eyedrop formulations.

The drug to be used in the context of the present invention can be selected from all drugs suitable for ophthalmic use and incorporation into the biocompatible polymer(s) as used (see above). Preferred are drugs selected from an antibiotic, such as levofloxacin, ciprofloxacin, gentamicin, an as above inflammatory, such as dexamethasone as well as other drug classes for ophthalmic use, such as prostaglandin analogues, beta-blockers, cholinergic agonists, carbonic anhydrase inhibitors, $\alpha$2-agonists, antihistamines, mastocyte stabilizers, wound healing agents, antibodies, gene therapeutics, virostatic agents, vasoconstrictors, analgesics, local anesthetics as well as diagnostics, and combinations thereof, in particular gentamicin and dexamethasone.

By dissolving drugs in the polymer solution prior to electrospinning, the generated polymer fibers become drug delivery systems with scalable encapsulation efficiency. The drug load can easily be adjusted by modifying either the drug concentration in the polymer solution or the thickness of the fiber mats, adapting the dosage to various clinical needs.

Advantageously, by incorporating drugs into a solid polymer fiber mat, the physico-chemical stability is increased, reducing the risk of oxidation, hydrolysis or phase separation exhibited by fluid or semi-solid dosage forms. Furthermore, the solid system is not susceptible to microbial contamination.

Another aspect of the present invention then relates to a pharmaceutical composition, comprising an effective amount of the drug-containing fiber mats according to the present invention, together with at least one ophthalmologically acceptable carrier or excipient. The pharmaceutical composition can be in separate compartment, for example one containing the fiber mats of the present invention, and the other the at least one ophthalmologically acceptable carrier or excipient. Furthermore, suitable applicators can be included.

Preferred is a pharmaceutical composition in the form of solid eye drops or an ocular dressing, in particular for the topical administration of combinations involving preferably water soluble and poorly water soluble drugs for the treatment of ophthalmologic diseases as well as healing of cornea defects and post-surgical recovery.

By dissolving drugs in the polymer solution prior to electrospinning, the generated polymer fibers become drug delivery systems with scalable encapsulation efficiency. The drug load can easily be adjusted by modifying either the drug concentration in the polymer solution or the thickness of the fiber mat, adapting the drug load to various clinical needs.

Advantageously, by incorporating drugs into a solid polymer fiber matrix or mat, the physico-chemical stability is increased, reducing the risk of oxidation, hydrolysis or phase separation exhibited by fluid or semi-solid dosage forms. Furthermore, the solid system is not susceptible to microbial contamination.

In contrast to eye drops or semisolid dosage forms, the inventor's system ensures a high dosing accuracy, easy application, and physico-chemical stability of the incorporated actives. Upon application, the polymers dissolve and thus prolong the contact time, without impairing the vision. The incorporable drugs and dosages can individually be adjusted to clinical needs by modifying the solvent of the polymer solution used for electrospinning. The unique features of the inventor's solid eye drops are favorable for the topical administration of a multitude of water soluble and poorly water soluble drugs for the treatment of ophthalmologic diseases as well as healing of cornea defects and post-surgical recovery.

The pharmaceutical composition of (or for use with) the invention is, typically, formulated to be compatible with its intended route of administration, here ocular. As used herein the term "ophthalmologically acceptable" excipients are intended to include any and all solvents, solubilizers, fillers, stabilizers, binders, plasticizers, absorbents, acids and bases, buffering agents, lubricants, controlled release excipients, diluents, emulsifying agents, humectants, dispersion media, coatings, preservatives, isotonic and absorption delaying agents, and the like, compatible with the intended pharmaceutical administration. The use of such media and agents for drugs is well-known in the art. Except insofar as any conventional media or agent is incompatible with the active compound and the ophthalmic use, use thereof in the compositions is contemplated. Supplementary agents can also be incorporated into the compositions.

In the context of the present invention, the term "effective amount" shall mean the amount of drug(s) in the composition as administered to a subject that produces a (desired) biological response. This is generally defined by the range between the minimum effective dose (MED) and the maximum tolerated dose (MTD). The MED is defined as the lowest dose level of a pharmaceutical product that provides a clinically significant response in average efficacy, which is also statistically significantly superior to the response provided by the placebo. Similarly, the MTD is the highest possible but still tolerable dose level with respect to a pre-specified clinical limiting toxicity. In general, these limits refer to the average patient population. Thus, the person of skill will be readily able to identify such amount (see also below), in particular for use of the composition in the prevention or treatment of ophthalmologic diseases, such as conjunctivitis, infectious conjunctivitis, glaucoma, and/or age-related macular degeneration as well as healing of cornea defects, e.g. injuries, and post-surgical recovery.

Another aspect of the present invention then relates to drug-containing fiber mats according to the present invention or the pharmaceutical composition according to the present invention for use in the prevention or treatment of diseases. Preferred are drug-containing fiber mats according to the present invention or the pharmaceutical composition according to the present invention for use in the prevention or treatment of ophthalmologic diseases, such as such as conjunctivitis, infectious conjunctivitis, glaucoma, and/or age-related macular degeneration as well as healing of cornea defects, e.g. injuries, and post-surgical recovery. Preferred is the pharmaceutical composition for use according to the present invention in the form of solid eye drops or an ocular dressing.

In another preferred aspect of the present invention, the above object is solved by a method for preventing or treating of an ophthalmologic disease, such as conjunctivitis, infectious conjunctivitis, glaucoma, and/or age-related macular degeneration as well as healing of cornea defects, e.g. injuries, and post-surgical recovery, comprising providing to a subject in need thereof an effective amount of the pharmaceutical composition according to the present invention as above.

Compounds (drugs and excipients) that are suitable and/or suitable for prevention or therapy are as disclosed above. The pharmaceutical composition for use in the invention may comprise combination therapeutics in accordance with the herein disclosed treatments, or alternatively, the invention may comprise a combination of pharmaceutical compositions, wherein each pharmaceutical composition comprises one single component of the combination therapeutics as described. As mentioned above, the compositions of the invention in this embodiment may be combined into one therapeutic kit.

The inventive system immediately dissolves upon application and increases the viscosity of the tear film, thus prolonging the contact time of the embedded actives and thus their pharmacological effect period. The system can easily be spread across the eye ball, thus reaching the periphery and ensuring even drug distribution, while minimizing the visual impairment

The composition of the invention is suitably administered to the patient at one time or over a series of treatments. If such composition of the invention and/or pharmaceutical composition is administered over a series of treatments, the total number of administrations for a given course of treatment may consist of a total of about 2, 3, 4, 5, 6, 7, 8, 9, 10 or more than about 10 treatments. For example, a treatment may be given once every day (or 2, 3 or 4 times a day) for a week, a month or even several months/years. In certain embodiments, the course of treatment may continue indefinitely, or until no more needed.

As used herein, a "subject" includes all mammals, including without limitation humans, and also includes dogs, cats, horses, sheep, goats, cows, rabbits, pigs and rodents (such as mice and rats). It will be appreciated that a particularly preferred subject according to the invention is a human subject, such as a human suffering from (or at risk of suffering from) an ocular disorder, disease or condition, for example a human patient.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein. Unless context dictates otherwise, the descriptions and definitions of the features set out above are not limited to any particular aspect or embodiment of the invention and apply equally to all aspects and embodiments which are described.

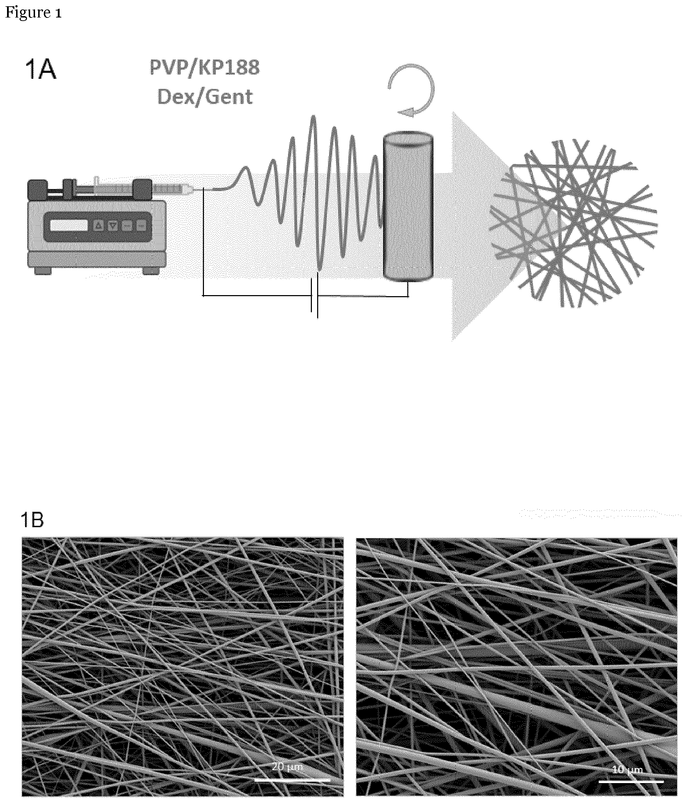

Electrospinning is highly challenging and dependent on a multitude of varying process parameters, such as polymer concentration and solution flow rate, applied voltage, collector rotation speed, as well as environmental factors (humidity, temperature). In order to obtain a homogeneous product, process steering plays an important role. The here developed formulation is characterized by a predictable and stable process, yielding in homogeneous and uniform fiber deposition (Figure 1A).

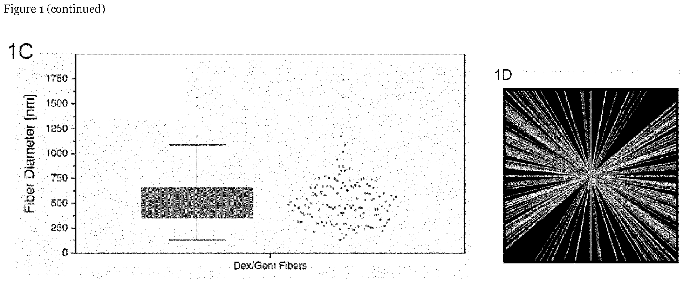

Fiber diameter and surface morphology of the resulting fiber mat are of major interest, because they directly influence the porosity of the scaffold. The obtained fiber mats were visualized with scanning electron microscopy (SEM). The acquired images revealed defect-free fibers with a smooth outer surface (Figure 1B). The average fiber diameter was determined to be 516.1 nm ± 237 nm (Figure 1C). Uniform fibers in nanometer size contribute to highly porous electrospun scaffolds. Such scaffolds exhibit a high surface-to-volume ratio, which leads to a faster disintegration of the formulation and release of incorporated APIs.

In order to quantify the porosity of the scaffold, the density of the fiber mat was determined by using measured dimensions and weight. The apparent density of the fiber mat was divided by the density of PVP and resulted in a porosity of 86.85% [Eq. 1].

$$Porosity \ [\%] = \left(1 - \frac{\rho_{apparent}}{\rho_{PVP}}\right) * 100\%$$

$$= \left(1 - \frac{0.1578 \ ^g/_{cm^3}}{1.2 \ ^g/_{cm^3}}\right) * 100\% = 86.85\% \qquad \textbf{(Eq.1)}$$

During the electrospinning process, the fibers undergo extensive whipping action, resulting in desired randomly aligned fiber deposition. The data obtained from SEM imaging, regarding fiber diameter and deposition angle were used to visualize the random fiber alignment with a common fiber intersection point (Figure ID). The line thickness and orientation in the visualization corresponds to the respective relative fiber diameter. The randomly aligned fibers are a key feature of electrospun fiber mats, responsible for the uniform mechanical properties in all stress directions.

In amorphous solid dispersions, drug molecules are molecularly dispersed within a solid matrix. For electrospun fibers, the API is dissolved in a polymer solution, which solidifies during the process, thus encapsulating the APIs. After dissolution of the polymer matrix, the incorporated APIs are molecularly released and solubilized within the surrounding fluid. Electrospun nanofibers can serve as an amorphous solid dispersion by overcoming drug recrystallization within the fibers. Thermal analysis can be used for the determination of the molecular structure of the resulting nanofibers.

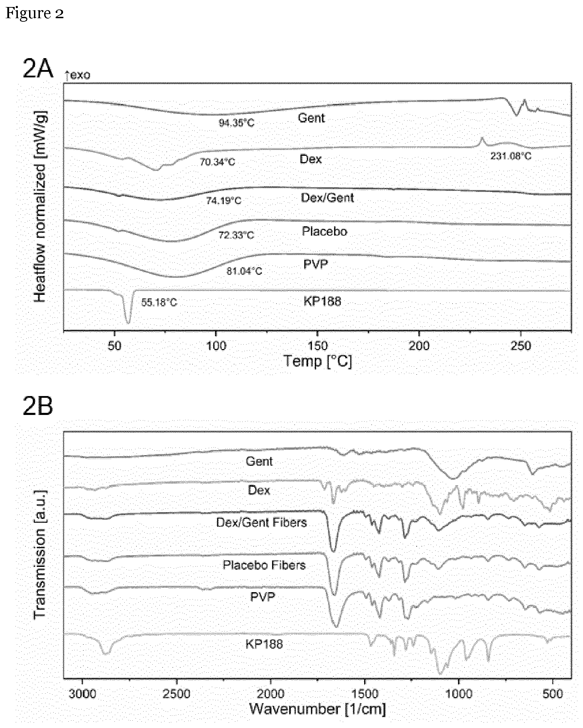

Thermograms show a melting point of poloxamer 188 (KP188) at 55.36°C (22). Measuring PVP, revealed a large endothermic event between 50°C and 110°C and with a peak at 81.04°C (Figure 2A). This endothermic event results from the loss of water from the hydrophilic polymer (23,24). The endothermic event could also be observed for the placebo and dexamethasone/gentamicin fibers. Gentamicin showed an additional melting point at 234°C and the rapid onset of thermal degradation (25-27). Dexamethasone had an exothermic event occurring at 231.08°C which correlates to the decomposition of the substance while melting (28). The water loss occurred for dexamethasone and gentamicin resulting in large endothermic events. Both fiber scaffolds did not show any thermal events attributed to the melting of crystalline APIs. The incorporation of dexamethasone and gentamicin into the PVP/poloxamer fibers leads to the formation of a solid system, in which the APIs are molecularly dispersed. This amorphous solid dispersion is characterized by its good miscibility between drugs and polymers. This fibrous drug delivery system could also increase the solubility and dissolution rates of BCS class IV drugs with a low solubility and bioavailability with the advantage of having a stable solid formulation without having disadvantages of coarsely dispersed particles in eye drops or in ointments. ATR-IR spectroscopy was used to determine the presence of both polymers within the fibers (Figure 2B). Distinct peaks at 1660 cm$^{-1}$ and 1285 cm$^{-1}$ can be attributed to PVP and to KP188, respectively. Multiple spectra recordings confirmed that both polymers are homogeneously distributed in the fiber mat.

One of the biggest challenges in developing advanced dosage forms for the ophthalmic application is to ensure a prolonged contact time on the ocular surface. The current standard therapy are eye drops, which have a short contact time on the ocular surface, due to low viscosity and high application volume, leading to rapid discharge and nasolacrimal drainage. To overcome this challenge, many eye drop formulations use viscosity enhancing excipients and gelling agents, which increase contact time. Nevertheless, these adjustments do not address the problems associated with excessive application volumes.

Advantageously, the here developed electrospun scaffold or mat consists of a water soluble polymer matrix, which immediately dissolves on the ocular surface. Upon this dissolution a highly viscous, fluid polymer network is established with the tear fluid, enhancing the ocular residence time of the API.

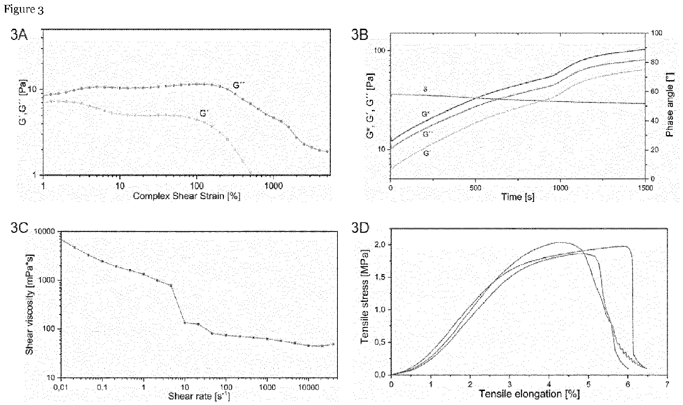

Oscillatory measurements were carried out to analyze the visco-elastic behavior of the resulting polymer fluid. The amplitude sweep measurement revealed a linear visco-elastic region up to 20% of the applied shear strain (Figure 3A). Measurements above this shear strain lead to a disruption of the inner polymer structure. All further experiments were carried out with a shear strain of 10% to ensure a non-destructive deformation.

Subsequently, oscillatory measurements over time were conducted. For high molecular weight polymers, like PVP (1,300,000 g/mol), three different states can occur regarding elastic modulus (G'), and loss modulus (G"). G'>>G" for chemically crosslinked systems, G'>G" for networks consisting of secondary bonds and G'<G" for polymer solutions, which are physically entangled (29). In this case, the formed polymer network revealed a loss modulus (G") greater than the elastic modulus (G') (Figure 3B), characteristics of a viscous-dominant (liquid-like) fluid. In contrast, if the elastic modulus (G') was greater compared to the loss modulus (G"), the immersed scaffold would exhibit an elastic-dominant (gel-like) behavior. In accordance to previous results, the phase angle tan (δ) >45° indicates viscous-dominant behavior.

Figure 3B shows an increase of the complex modulus (G*) over time, as well as its components, the elastic modulus (G') and loss modulus (G") (Eq. 2).

$$G^* = G' + G'' \qquad \textbf{(Eq.2)}$$

This can be explained due to reformation its internal structure. Although all three moduli are increasing, no intersection between the elastic and loss modulus can be observed, thus the resulting eye drop remain viscous-dominant.

The frequency sweep measurement describes the time-dependent behavior of a sample in a non-destructive deformation range. Low oscillation frequencies describe polymers at rest (closed eye) while high frequencies describe polymers subject to fast motions. Measurements of the dissolved fiber matrix revealed that its viscous-dominant state is time-independent and occurs at high as well as low shear rates. "Weak" gels would show a crossover at the low frequency because they start flowing as a high viscosity liquid there, while real gels (e.g. crosslinked systems) have a greater elastic modulus at all times, even at low frequencies (30).

Mimicking of the human eyelid movement

Another important aspect is the flow behavior in high shearing conditions, mimicking the blinking process. In these high shear condition, the resulting polymer network has to undergo a shear thinning to reduce the foreign body sensation in the eye. When blinking, the shear rate exhibited on the formulation is between 4,000 s$^{-1}$ and 30,000 s$^{-1}$ (31).

At shear rates greater 40 s$^{-1}$, the systems first undergo extensive shear thinning and then remains as low as 47 mPa*s, which is 24 fold higher compared to the viscosity of tear fluid (Figure 3C). Even in high shear conditions, the viscosity is sufficiently high to ensure a long contact time to the ocular surface.

Dissolving the electrospun fibers in simulated tear fluid resulted in visco-elastic fluid of viscous-dominant type. At shear rates mimicking the blinking process, the formulation showed shear thinning. The chosen polymers lead to an increased ocular residence time of the APIs and the viscosity adapts to the blinking process, reducing the nasolacrimal drainage and foreign body sensation in the eye.

Fiber mat thickness and mechanical characterization

The fiber mat must be sufficiently tough in order to prevent rupture during post-manufacture processes (e.g. cut out, packaging), transport, storage, as well as application on the ocular surface. For the fiber formulations, the average fiber mat thickness was approx. 150 $\mu$m. Upon stretching, the fiber mat exhibits elastic properties up to elongations of approx. 4%, before the fibers show plastic deformation. Following plastic deformation, the specimens rapidly enter a stage of cracking. Specimens with a size of 8 mm x 42 mm x 0.15 mm (w x 1 x d) withstood maximum forces of 2.22 $\pm$ 0.024 N, equivalent to approximately 200 g or 2 MPa (N/mm$^2$). Young's modulus is the increase of slope in the elastic region of the stress strain curve and describes the resistance to elastic deformation. A higher Young's modulus indicates a more brittle and stiff material. The modulus of the tested electrospun scaffold was 77.32 $\pm$ 4.67 MPa. The exhibited mechanical properties (e.g. Young's modulus, ultimate tensile strength) are outperforming those of different soft contact lenses that were successfully marketed (32, 33). The electrospun fiber mats withstand high tensile forces, making ophthalmic application feasible without rupture and can be handled like soft contact lenses.

Quantification of dexamethasone and gentamicin

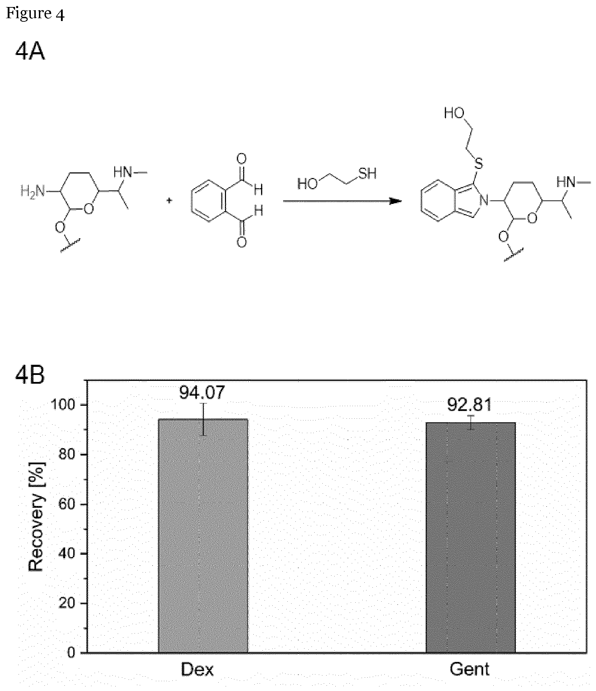

Solid eye drops need to provide an immediate and complete release of the encapsulated drugs. The fibers dissolve immediately when in contact with fluid. The drug content of a single dose was determined for dexamethasone as well as gentamicin. Dexamethasone can be determined using reverse phase high performance liquid chromatography (RP-HPLC), while gentamicin can be quantified using a colorimetric detection reaction. The underlying colorimetric reaction mechanism with o-phthaldialdehyde (OPA) is shown in Figure 4A.

Both methods show correlation coefficients $R^2$ > 0.998 and can thus be used for the quantification of the respective compound. The recovery of the compounds is determined according to the following equation, whereas the theoretical mass is determined by weight of the compounds within the dry formulation (Eq.2). The primary amine groups of gentamicin are reacting with OPA and 2-mercaptoethanole to form a fluorescent product (17). Gentamicin load in a 6 mm circular solid eye drop was determined to be 49.65 $\pm$ 0.49 $\mu$g (recovery 92.81% $\pm$ 2.85%) and dexamethasone 8.97$\pm$0.10$\mu$g (recovery 94.07% $\pm$ 6.56%) (Figure 4B). The release of the dexamethasone and gentamicin was quantitative and immediate. The drug load can be adjusted to various clinical dosage needs by increasing the drug load in the electrospinning solution, by changing the size of the solid eye drops or by increasing the thickness of the fiber mats during processing.

$$Recovery\ (\%) = \frac{m_{experimental}}{m_{theoretical}} \qquad \textbf{(Eq.3)}$$

Antibacterial activity and drug stability

The antimicrobial activity of the incorporated API (i.e. gentamicin) can be determined by disk diffusion analysis and confirms, whether the electrospinning process had an impact on the biological activity. Assessment of antimicrobial activity was performed, using two prevalent pathogens associated with bacterial conjunctivitis (*Staphylococcus aureus* and *Staphylococcus epidermidis*). *S. epidermidis* is also named as one of the pathogens for determination of microbiological enumeration for gentamicin in the European pharmacopoeia ("Microbiological assay of antibiotics", Ph.Eur. 2.7.2).

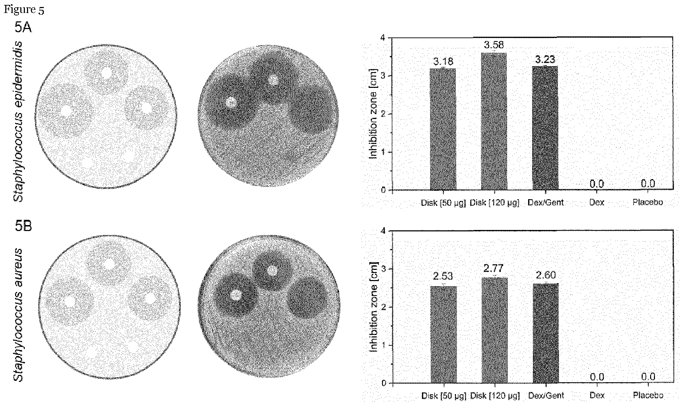

*S. epidermidis* was shown to be susceptible to the dex/gent fiber mat (containing 50 $\mu$g gentamicin), as well as to both gentamicin disks (50 $\mu$g, 120 $\mu$g gentamicin). The inhibition zone diameters correlate with the drug load and no significant difference could be observed between the electrospun drug delivery systems and the standard (Figure 5A). No visible inhibition zone could be detected for placebo fibers or dex fibers, leading to the conclusion that the antibacterial effect is limited to the released gentamicin. *S. aureus* showed comparable results although it had a smaller inhibition zone compared to S. *epidermidis,* which is consistent with the literature (34, 35) (Figure 5B).

The fiber mat exhibited the same antimicrobial effect as the impregnated gentamicin disk (50 µg gentamicin) and had a slightly smaller inhibition zone compared to the commercially available gentamicin disk (120 µg gentamicin). During the electrospinning process, the antimicrobial activity was maintained, leading to drug stabilization in the matrix. The used polymers and dexamethasone in the fiber matrix did not influence the growth of the bacteria strains used. Stability testing in week 4 and 12 after production confirmed unaltered antimicrobial activity with no significant loss in potency.

The inventors have successfully developed a polymer-based solid eye drop formulation, which rapidly dissolves upon application on the ocular surface. These systems are manufactured by electrospinning, a process for generating polymer fibers with diameters in the nanometer range, which form fiber mats. These fiber mats have a high surface-to-volume ratio, leading to their instant dissolution upon contact with the tear fluid. This versatile platform technique is capable integrating and releasing various molecularly dispersed APIs onto the ocular surface.

The electrospun scaffold consists of two FDA approved polymers, which increase the viscosity of the tear film after application. This leads to a prolonged contact time on the ocular surface, compared to fluid, low viscosity eye drops. By including two APIs suitable for treatment of bacterial conjunctivitis (i.e. gentamicin sulfate and dexamethasone phosphate), the inventors achieved to design and produce a promising drug delivery system for ocular delivery of actives.

The system is capable of achieving precise dosing of both active ingredients, which were quantified after release from the fibers. The stabilizing effect of the formulation on the incorporated actives during storage was assessed by antibacterial activity testing over the estimated shelf life period, using two prevalent pathogens for bacterial conjunctivitis. The nanofibers released the complete amount of active substances and the antibiotic exhibited full antimicrobial effect. Experiments following OECD guidelines are currently performed to determine corneal irritation of the inventor's formulation (12).

In contrast to eye drops or semisolid dosage forms, the inventor's system ensures a high dosing accuracy, easy application, and physico-chemical stability of the incorporated actives. Upon application, the polymers dissolve and thus prolong the contact time, without impairing the vision. The incorporable drugs and dosages can individually be adjusted to clinical needs by modifying the solvent of the polymer solution used for electrospinning. The unique features of the inventor's solid eye drops are favorable for the topical administration of a multitude of water soluble and poorly water soluble APIs for the treatment of ophthalmologic diseases.

In summary, the inventors have designed a nanofibrous ocular drug delivery system for co-delivery of dexamethasone and gentamicin by electrospinning biocompatible polymers. The unique feature of this system is its highly porous structure and great surface to volume ratio. This leads to rapid dissolution of the drug delivery system upon application, releasing molecularly dispersed API into the tear fluid, without adding any volume to this low volume body compartment. The used polymers immediately increase the viscosity of the tear fluid, thus prolonging contact time. Compared to established drug delivery systems, the formulation ensures a high dosing accuracy and API recovery. Gentamicin released from the fibers inhibited the growth of disease-specific bacteria, and showed full antibacterial activity in disc diffusion analysis over a 12-week shelf life period. Testing of the drug delivery system on an in vitro cornea model validated full biocompatibility. The solid eye drops developed in this study, have a great potential as a platform technology for ocular drug delivery systems for various clinical needs. These systems are highly suitable for hydrophilic actives as shown in this study, but also allow the integration of lipophilic APIs, upon solvent adjustment. By overcoming the challenges associate with established ocular drug delivery systems, this formulation is highly promising in solving current and future clinical drug delivery needs.

The present invention will now be described further in the following examples with reference to the accompanying Figures, nevertheless, without being limited thereto. For the purposes of the present invention, all references as cited herein are incorporated by reference in their entireties.

Figure 1 shows a schematic illustration of the electrospinning process (1A). SEM images were acquired after gold sputter-coating (1B) Fiber diameter distribution (1C) was evaluated based on SEM visualization (n = 150, average = 516.1 nm, block shows 25-75 percentile). 1D depicts the orientation of the fibers within x-y direction with a common central crossing point. Line diameters correlate with the fiber diameters of the respective fibers.

Figure 2 shows thermograms (2A) and ATR-IR spectra (2B) for polyvinylpyrrolidone (PVP), poloxamer 188 (KP188), dexamethasone-21-phosphate (dex), gentamicin sulfate (gent) as well as for dex/gent and placebo fibers.

Figure 3 shows results of oscillatory viscometry that was performed for the determination of the linear-viscoelastic region (3A) and to measure the change of viscosity over time (3B). The human eyelid movement was mimicked and assessed by rotational viscometry (3C). The observed mechanical properties of the fiber mat are shown as stress-strain curves (3D).

Figure 4 shows the reaction mechanism of o-phthaldialdehyde (OPA) with a primary amino group of gentamicin to form a compound, which can be quantified by fluorimetry (4A). The drug load recovery of the nanofibers was quantified using RP-HPLC and fluorimetry (4B).

Figure 5 shows inhibition zones of commercially available disks (120 μg), self-impregnated disks (50 μg), dex/gent fibers, dex fibers and placebo fibers are shown for staphylococcus epidermidis (5A) and staphylococcus aureus (5B). The diameter of the inhibition zone for the specific bacterial culture is shown in the column chart.

EXAMPLES

**Materials and chemicals**

Dexamethasone 21-phosphate disodium salt (further referred to as dexamethasone) and polyvinylpyrrolidone (PVP, 1,300,000 g/mol), o-phthaldialdehyde (OPA), 2-mercaptoethanol and boric acid were obtained from Sigma-Aldrich (Darmstadt, Germany). Gentamicin sulfate (further referred to as gentamicin) was purchased from Fagron (Glinde, Germany). Methanol, ethanol, Difco™ Nutrient Agar, and sodium hydroxide pellets were obtained from VWR Chemicals (Darmstadt, Germany). *Staphylococcus aureus subsp. aureus* (ATCC 25923) and *Staphylococcus epidermidis* (ATCC 12228) (CultiLoop) as well as 120 μg gentamicin disks were purchased from Thermo Fisher Scientific (Dreieich, Germany). Sodium chloride, sodium hydrogencarbonate and calcium chloride (anhydrous), were purchased from Merck KgaA (Darmstadt, Germany). Kolliphor P188 was kindly provided by BTC Europe (Monheim, Germany). All chemicals were used without further purification. All other chemicals used were at least analytical grade.

**Preparation of electrospinning solutions**

PVP (15% w/v) and poloxamer 188 (2.5% w/v) were dissolved in a mixture of double distilled water/ethanol in a ratio of 4:1 (v/v) under stirring conditions at room temperature. After complete dissolution, gentamicin (1.4% w/v) and dexamethasone (0.28% w/v) were added. After complete dissolution, the solution was briefly degassed in an ultrasonic bath. The polymer solution was then transferred into a 5 mL single-use syringe (B. Braun, Melsungen, Germany), fitted with a blunt needle tip (27G, B. Braun, Melsungen, Germany).

**Electrospinning**

The formulation was electrospun in a custom-built electrospinning cabinet, capable of regulating temperature and moisture. The solution was electrospun with a flow rate of 2.0 mL/h at 15 kV onto a rotating drum cylinder with a diameter of 10 cm at 100 revolutions per minute (RPM). Temperature was set to 21°C and moisture to 20% relative humidity, respectively. In addition to these fibers (further referred to as dex/gent fibers), two control scaffolds were electrospun and used as controls. The first control was generated without APIs (further referred to as placebo fibers) and the second control only containted dexamethasone as API (further referred to as dex fibers). After compfletion, the electrospun fiber mats were stored in a desiccator at ambient temperatures until further use.

**Scanning electron microscopy and fiber analysis**

Scanning electron microscopy (SEM) images were acquired at 5 kV on a field emission scanning electron microscope (S 4500, Hitachi, Chiyoda, Japan). Samples were gold-sputtered for three minutes before image acquisition (Agar Sputter Coater, Agar Scientific, Stansted, United Kingdom). The obtained images were analyzed using the software Fiji [12]. 150 fibers were randomly chosen and their diameter and orientation measured. The fiber orientation and relative diameter of the individual fibers was processed in a visualization grid using Microsoft Excel 2016.

**Attenuated total reflection-infrared (ATR-IR) spectroscopy**

Infrared spectra were recorded with an ALPHA II Platinum FT-IR spectrometer (Bruker, Billerica, MA, USA) utilizing OPUS-TOUCH software (Version 8.1). For each data point, twenty-four spectra at a resolution of 4 $cm^{-1}$ and over the shift range of 3100 - 400 $cm^{-1}$ were recorded, averaged, and normalized.

**Differential scanning calorimetry (DSC)**

A DSC 3 (Mettler Toledo, Columbus, OH, USA), equipped with an Intra-Cooler TC100MT (Peter Huber Kältemaschinenbau AG, Offenburg, Germany) was used to record thermograms from 25°C to 275°C with a heating rate of 10°C/min under nitrogen atmosphere (50 mL/min). Sample quantities of approximately 5 mg were used for each measurement. All samples were measured in cold-welded 40 $\mu$L aluminum sample pans against empty 40 $\mu$L aluminum pans. The obtained thermograms (mW/g) were normalized to the largest peak by using OriginPro 2018 (OriginLab Corporation, Northampton, MA; USA). The device was calibrated with indium.

**Rheological analysis**

Rheological properties were assessed with a KinexusLab+ (Netzsch, Selb, Germany) equipped with a 20 mm plate-plate geometry and gap distance was set to 50 $\mu$m. 12 mm diameter circular fiber samples were cut with a biopsy punch (PicuPunch, Xiomedics, Unterhaching, Germany) and immersed in 40 $\mu$L of simulated tear fluid (100 mL double distilled water contained 0.2 g $NaHCO_3$, 0.67 g NaCl and 0.06 g $CaCl_2$ [13]). The pH was adjusted to 7.4 by adding sodium hydroxide. All measurements were carried out at the respective ocular surface temperature of 34°C (14, 15) with a passive solvent trap.

Amplitude sweep measurements from 1% to 10,000% were performed to determine the maximum applicable strain amplitude within the linear viscoelastic region (LVER). Further measurements were carried out within LVER. Oscillation experiments were used to determine the stress response of the sample to a sinusoidally varying strain over a period of 25 minutes. The experiment was carried out with a frequency of 1 Hz and a shear strain of 10%. Frequency sweep measurements were carried out in the range between 0.1 Hz to 20 Hz to determine the time-dependent behavior. Rotational viscometry was carried out in triplicate between a shear rate of 0.01 $s^{-1}$ and 40,000 $s^{-1}$, mimicking the blinking of the human eye, to determine shear thinning [16].

**Fiber mat thickness analysis**

The fiber mat thickness was measured using a MiniTest 735 with an F5 sensor (ElektroPhysik Dr. Steingroever GmbH & Co. KG, Cologne, Germany). The fibers were placed on stainless steel and covered with a tared microscopy cover slip. The sensor was then placed on top of the glass slip. Measurements were carried out in fivefold determination. The results were used as input parameters for tensile testing measurements.

**Tensile strength analysis**

Mechanical fiber properties were assessed on a vertical single-column tensile testing system (Model 5942, Instron, Norwood, MA, USA). Scaffolds were cut evenly into rectangular shape with the size of 0.8 cm x 4.2 cm (width x length) and clamped with rubber jaw faces into pneumatic grips. A 50 N load cell was used for all experiments. Before recording stress strain curves, 50 mN pre-stress was applied to each sample, ensuring to test the specimen in a reproducible manner. Mechanical testing was then carried out with a speed of 10 mm/min, distance and force were recorded simultaneously. The analysis was conducted with Bluehill Universal software (Instron, Norwood, MA, USA). For mechanical characterization, Young's modulus, tensile elongation and stress, and ultimate tensile strength were assessed. All experiments were carried out in threefold determination.

**Quantification of dexamethasone 21-phosphate disodium**

HPLC analysis was performed on a Dionex UltiMate 3000 (Thermo Fisher Scientific, Dreieich, Germany) with a temperature-controlled autosampler (25°C), using a 100 mm RP-18 column (BDS Hypersil C18, 100 x 4.6 mm, 3 $\mu$m particle size, Thermo Fisher Scientific, Dreieich, Germany) at ambient temperature (25°C).

An isocratic method was used (25% (v/v) Ethanol, 75% (v/v) double distilled water) at a constant flow rate of 0.6 mL/min. Chromatograms were recorded with a diode array detector (DAD) at 240 nm and evaluated in Chromeleon 7 (Thermo Fisher Scientific, Dreieich, Germany). A calibration was carried out from 0 - 1,000 $\mu$g/mL ($R^2$ = 1).

**Quantification of gentamicin sulfate**

Boric acid (6.175g) was dissolved in 200 mL double distilled water, the pH was adjusted to 10.4 by adding sodium hydroxide solution (25% w/v).

O-phthaldialdehyde (OPA) reagent was freshly prepared by dissolving OPA (0.2g) in 1 mL methanol and adding 19 mL of the boric acid buffer (pH 10.4). Subsequently, 0.4 mL of

2-mercaptoethanol was added to the solution and the pH was adjusted to 10.4 with sodium hydroxide solution (17). A master mix was prepared by adding 1.125 mL of the freshly OPA reagent to 13.875 mL methanol.

Three 6 mm circular fiber mat punches were dissolved in 1 mL double distilled water. A calibration was performed from 1 - 30 $\mu$g/mL in double distilled water. The determination of samples and calibration was carried out in threefold. 100 $\mu$L of the testing and calibration solutions were transferred to a 96-well microplate for fluorescence-based assays, supplemented with 100 $\mu$L OPA master mix. The solution was incubated for 10 minutes at room temperature in the dark. The measurement was carried out in a ClarioStar Plus plate reader (BMG Labtech, Ortenberg, Germany) at an excitation wavelength of 340 nm and an emission wavelength of 455 nm.

## Disc diffusion analysis

To evaluate the antibacterial efficacy of the electrospun scaffolds, disc diffusion method was performed in accordance with the EUCAST (European Committee on Antimicrobial Susceptibility Testing) guidelines. *Staphylococcus epidermidis* (ATCC 12228) and *Staphylococcus aureus subsp. aureus* (ATCC 25923) were used as test organisms. Single colonies were dispersed in sterile PBS obtaining a turbidity equivalent to that of 0.5 McFarland standard. Afterwards, the dispersion was spread over Mueller Hinton agar plates.

Dex/gent fibers were cut into circular discs with 6 mm in diameter, containing approximately 50 $\mu$g of gentamicin sulfate (determined according to 3.11). Dex fibers and placebo fibers were processed equally. As standards, next to commercial gentamicin discs (120 $\mu$g), discs containing 50 $\mu$g gentamicin were prepared by pipetting 2.5 $\mu$L of a 20 $\mu$g/$\mu$L gentamicin sulfate stock solution onto 6 mm circular filter membranes. All samples were placed on agar surface and incubated at 37°C for 24 hours. The inhibition zone was measured with a ruler with adequate scale. Images were recorded with a Canon EOS M50 (Canon, Tokyo, Japan). For stability testing of gentamicin in the fiber mats, experiments were carried out at 0, 4 and 12 weeks after electrospinning. All experiments were carried out in triplicate.

## Statistical analysis

All results presented as their mean $\pm$ standard deviation (SD). Statistically significant differences between experimental groups were analyzed using the Wilcoxon signed-rank test, which requires no normality of the data and is specifically applied for small data sets. P-values < 0.05 were considered to be statistically significant and indicated by (*, p < 0.01 **). All calculations were performed using Microsoft Office Excel 2016 and Origin Pro 2018.

## References

1. A.A. Azari, N.P. Barney, Conjunctivitis: a systematic review of diagnosis and treatment, JAMA 310 (2013) 1721-1729. https://doi.org/10.1001/jama.2013.280318.
2. A.F. Smith, C. Waycaster, Estimate of the direct and indirect annual cost of bacterial conjunctivitis in the United States, BMC Ophthalmol. 9 (2009) 13. https://doi.org/10.1186/1471-2415-9-13.
3. D. Achouri, K. Alhanout, P. Piccerelle, V. Andrieu, Recent advances in ocular drug delivery, Drug Dev. Ind. Pharm. 39 (2013) 1599-1617. https://doi.org/10.3109/03639045.2012.736515.
4. K. Nayak, M. Misra, A review on recent drug delivery systems for posterior segment of eye, Biomed. Pharmacother. 107 (2018) 1564-1582. https://doi.org/10.1016/j.biopha.2018.08.138.
5. J.M. Tiffany, The normal tear film, Dev. Ophthalmol. 41 (2008) 1-20. https://doi.org/10.1159/000131066.
6. P.-L. Destruel, N. Zeng, F. Brignole-Baudouin, S. Douat, J. Seguin, E. Olivier, M. Dutot, P. Rat, S. Dufaÿ, A. Dufay-Wojcicki, M. Maury, N. Mignet, V. Boudy, In Situ Gelling Ophthalmic Drug Delivery System for the Optimization of Diagnostic and Preoperative Mydriasis: In Vitro Drug Release, Cytotoxicity and Mydriasis Pharmacodynamics, Pharmaceutics 12 (2020). https://doi.org/10.3390/pharmaceutics12040360.
7. N. Mittal, G. Kaur, In situ gelling ophthalmic drug delivery system: Formulation and evaluation, J. Appl. Polym. Sci. 131 (2014) n/a-n/a. https://doi.org/10.1002/app.39788.
8. Y. Qian, F. Wang, R. Li, Q. Zhang, Q. Xu, Preparation and evaluation of in situ gelling ophthalmic drug delivery system for methazolamide, Drug Dev. Ind. Pharm. 36 (2010) 1340-1347. https://doi.org/10.3109/03639041003801893.
9. W. Kircher, Proper administration of topical ophthalmics Augenarzneimittel korrekt angewandt.
10. P. Saarinen-Savolainen, T. Järvinen, K. Araki-Sasaki, H. Watanabe, A. Urtti, Evaluation of cytotoxicity of various ophthalmic drugs, eye drop excipients and cyclodextrins in an immortalized human corneal epithelial cell line, Pharmaceutical research 15 (1998) 1275-1280. https://doi.org/10.1023/a:1011956327987.
11. A. Kumari, P.K. Sharma, V.K. Garg, G. Garg, Ocular inserts - Advancement in therapy of eye diseases, J. Adv.

Pharm. Technol. Res. 1 (2010) 291-296. https://doi.org/10.4103/0110-5558.72419.

12. J. Schindelin, I. Arganda-Carreras, E. Frise, V. Kaynig, M. Longair, T. Pietzsch, S. Preibisch, C. Rueden, S. Saalfeld, B. Schmid, J.-Y. Tinevez, D.J. White, V. Hartenstein, K. Eliceiri, P. Tomancak, A. Cardona, Fiji: an open-source platform for biological-image analysis, Nat. Methods 9 (2012) 676-682. https://doi.org/10.1038/nmeth.2019.

13. M.R.C. Marques, R. Loebenberg, M. Almukainzi, Simulated Biological Fluids with Possible Application in Dissolution Testing, Dissolution Technol. 18 (2011) 15-28. https://doi.org/10.14227/dt180311p15.

14. J.P. Craig, I. Singh, A. Tomlinson, P.B. Morgan, N. Efron, The role of tear physiology in ocular surface temperature, Eye (Lond) 14 (Pt 4) (2000) 635-641. https://doi.org/10.1038/eye.2000.156.

15. C. Purslow, J.S. Wolffsohn, Ocular surface temperature: a review, Eye & contact lens 31 (2005) 117-123. https://doi.org/10.1097/01.icl.0000141921.80061.17

16. P.-L. Destruel, N. Zeng, J. Seguin, S. Douat, F. Rosa, F. Brignole-Baudouin, S. Dufaÿ, A. Dufay-Wojcicki, M. Maury, N. Mignet, V. Boudy, Novel in situ gelling ophthalmic drug delivery system based on gellan gum and hydroxyethylcellulose: Innovative rheological characterization, in vitro and in vivo evidence of a sustained precorneal retention time, International Journal of Pharmaceutics 574 (2020) 118734. https://doi.org/10.1016/j.ijpharm.2019.118734.

17. J. Gubernator, Z. Drulis-Kawa, A. Kozubek, A simply and sensitive fluorometric method for determination of gentamicin in liposomal suspensions, 2006.

18. A. Greiner, J.H. Wendorff, Electrospinning: a fascinating method for the preparation of ultrathin fibers, Angew. Chem. Int. Ed Engl. 46 (2007) 5670-5703. https://doi.org/10.1002/anie.200604646.

19. H. Almeida, M.H. Amaral, P. Lobão, J.M. Sousa Lobo, Applications of poloxamers in ophthalmic pharmaceutical formulations: an overview, Expert Opin. Drug Deliv. 10 (2013) 1223-1237. https://doi.org/10.1517/17425247.2013.796360.

20. Y.C. Kim, M.D. Shin, S.F. Hackett, H.T. Hsueh, R. Lima E Silva, A. Date, H. Han, B.-J. Kim, A. Xiao, Y. Kim, L. Ogunnaike, N.M. Anders, A. Hemingway, P. He, A.S. Jun, P.J. McDonnell, C. Eberhart, I. Pitha, D.J. Zack, P.A. Campochiaro, J. Hanes, L.M. Ensign, Gelling hypotonic polymer solution for extended topical drug delivery to the eye, Nat. Biomed. Eng. 4 (2020) 1053-1062. https://doi.org/10.1038/s41551-020-00606-8.

21. A.A. Al-Kinani, G. Zidan, N. Elsaid, A. Seyfoddin, A.W.G. Alani, R.G. Alany, Ophthalmic gels: Past, present and future, Advanced Drug Delivery Reviews 126 (2018) 113-126. https://doi.org/10.1016/j.addr.2017.12.017.

22. M.A. Miranda, P.D. Marcato, I.P.S. Carvalho, L.B. Silva, D.L. Ribeiro, R. Amaral, K. Swiech, J.K. Bastos, J.A.R. Paschoal, R.B. Dos Reis, M.V.L.B. Bentley, Assessing the cytotoxic potential of glycoalkaloidic extract in nanoparticles against bladder cancer cells, J. Pharm. Pharmacol. 71 (2019) 1520-1531. https://doi.org/10.1111/jphp.13145.

23. M.A. da Costa, R.C. Seiceira, C.R. Rodrigues, C.R.D. Hoffmeister, L.M. Cabral, H.V.A. Rocha, Efavirenz dissolution enhancement I: co-micronization, Pharmaceutics 5 (2012) 1-22. https://doi.org/10.3390/pharmaceutics5010001.

24. D.-G. Yu, L.-M. Zhu, C.J. Branford-White, J.-H. Yang, X. Wang, Y. Li, W. Qian, Solid dispersions in the form of electrospun core-sheath polymer fibers, Int. J. Nanomedicine 6 (2011) 3271-3280. https://doi.org/10.2147/IJN.S27468.

25. M.S. Aw, S. Simovic, J. Addai-Mensah, D. Losic, Silica microcapsules from diatoms as new carrier for delivery of therapeutics, Nanomedicine (Lond) 6 (2011) 1159-1173. https://doi.org/10.2217/nnm.11.29.

26. P.O. Nnamani, F.C. Kenechukwu, E.U. Dibua, C.C. Ogbonna, U.L. Monemeh, A.A. Attama, Transdermal microgels of gentamicin, Eur. J. Pharm. Biopharm. 84 (2013) 345-354. https://doi.org/10.1016/j.ejpb.2012.11.015.

27. F. Shiehzadeh, M. Tafaghodi, M.-L. Dehghani, F. Mashhoori, B.S. Fazly Bazzaz, M. Imenshahidi, Preparation and Characterization of a Dry Powder Inhaler Composed of PLGA Large Porous Particles Encapsulating Gentamicin Sulfate, Adv. Pharm. Bull. 9 (2019) 255-261. https://doi.org/10.15171/apb.2019.029.

28. A. Karava, M. Lazaridou, S. Nanaki, G. Michailidou, E. Christodoulou, M. Kostoglou, H. Iatrou, D.N. Bikiaris, Chitosan Derivatives with Mucoadhesive and Antimicrobial Properties for Simultaneous Nanoencapsulation and Extended Ocular Release Formulations of Dexamethasone and Chloramphenicol Drugs, Pharmaceutics 12 (2020). https://doi.org/10.3390/pharmaceutics12060594.

29. J.D. Ferry, Viscoelastic properties of polymers, Third edition, John Wiley & Sons, New York, Chichester, Brisbane, Toronto, Singapore, 1980.

30. J.R. Robinson, G.M. Mlynek, Bioadhesive and phase-change polymers for ocular drug delivery, Advanced Drug Delivery Reviews 16 (1995) 45-50.

31. P.-L. Destruel, N. Zeng, J. Seguin, S. Douat, F. Rosa, F. Brignole-Baudouin, S. Dufaÿ, A. Dufay-Wojcicki, M. Maury, N. Mignet, V. Boudy, Novel in situ gelling ophthalmic drug delivery system based on gellan gum and hydroxyethylcellulose: Innovative rheological characterization, in vitro and in vivo evidence of a sustained precorneal retention time, International Journal of Pharmaceutics 574 (2020) 118734. https://doi.org/10.1016/j.ijpharm.2019.118734.

32. T.S. Bhamra, B.J. Tighe, Mechanical properties of contact lenses: The contribution of measurement techniques and clinical feedback to 50 years of materials development, Cont. Lens Anterior Eye 40 (2017) 70-81. https://doi.org/10.1016/j.clae.2016.11.005.

33. I. Tranoudis, N. Efron, Tensile properties of soft contact lens materials, Cont. Lens Anterior Eye 27 (2004) 177-191. https://doi.org/10.1016/j.clae.2004.08.002.

34. J.A. Schafer, L.B. Hovde, J.C. Rotschafer, Consistent rates of kill of Staphylococcus aureus by gentamicin over a 6-fold clinical concentration range in an in vitro pharmacodynamic model (IVPDM), J Antimicrob Chemother 58 (2006) 108-111. https://doi.org/10.1093/jac/dkl216.

35. V.H. Tam, S. Kabbara, G. Vo, A.N. Schilling, E.A. Coyle, Comparative pharmacodynamics of gentamicin against Staphylococcus aureus and Pseudomonas aeruginosa, Antimicrob. Agents Chemother. 50 (2006) 2626-2631. https://doi.org/10.1128/AAC.01165-05

**Claims**

1. A method for producing drug-containing polymer fibers for ocular use, comprising

   a) providing at least one drug suitable for ocular use,
   b) adding said at least one drug to a suitable solution of at least one biocompatible polymer, wherein said polymer is capable of incorporating and stabilizing the at least one drug, is hydrophilic, is tear fluid dissolvable and increases the viscosity of the tear fluid when brought in contact with said tear fluid, and
   c) electrospinning of said solution of biocompatible polymer of b) in order to produce said drug-containing polymer fibers.

2. A method for producing drug-containing fiber mats for ocular use, comprising the method according to claim 1, and further comprising the step of solvent evaporation and forming a fiber mats, preferably consisting of dried polymer polymer fibers.

3. The method according to claim 1 or 2, wherein said at least one biocompatible polymer is selected from polymers acceptable for ophthalmic use, such as, for example, polyvinylpyrrolidone, poloxamer, and combinations thereof.

4. The method according to any one of claims 1 to 3, wherein said polymer fibers are comprised in a solid formulation.

5. The method according to any one of claims 1 to 4, wherein said drug is selected from an antibiotic, such as levofloxacin, ciprofloxacin, gentamicin, an antiinflammatory, such as dexamethasone as well as other drug classes for ophthalmic use, such as prostaglandin analogues, beta-blockers, cholinergic agonists, carbonic anhydrase inhibitors, $\alpha$2-agonists, antihistamines, mastocyte stabilizers, wound healing agents, antibodies, gene therapeutics, virostatic agents, vasoconstrictors, analgesics, local anesthetics as well as diagnostics, and combinations thereof, in particular gentamicin and dexamethasone

6. Drug-containing polymer fibers or drug-containing fiber mat, produced according to a method according to any one of claims 1 to 5.

7. Drug-containing polymer fibers or drug-containing fiber mat according to claim 6, wherein the biocompatible polymer is a mixture of PVP and poloxamer 188.

8. Drug-containing polymer fibers or drug-containing fiber mat according to claim 6 or 7, wherein the at least one drug is a combination of gentamicin and dexamethasone.

9. Pharmaceutical composition, comprising an effective amount of the drug-containing polymer fibers or drug-containing fiber mat according to any one of claims 6 to 8, preferably in the form of solid eye drops.

10. The drug-containing polymer fibers or drug-containing fiber mat according to any one of claims 6 to 8 or the pharmaceutical composition according to claim 9 for use in the diagnosis, prevention or treatment of diseases, in particular for use in the diagnosis, prevention, or treatment of ophthalmologic diseases, such as conjunctivitis, infectious conjunctivitis, glaucoma, and/or age-related macular degeneration as well as healing of cornea defects, e.g. injuries, and post-surgical recovery.

11. The pharmaceutical composition for use according to claim 10 in the form of solid eye drops or an ocular dressing.

12. A method for diagnosing, preventing or treating of an ophthalmologic disease, such as conjunctivitis, infectious conjunctivitis, glaucoma, and/or age-related macular degeneration as well as healing of cornea defects, e.g. injuries, and post-surgical recovery, comprising providing to a patient in need thereof an effective amount of the pharmaceutical composition according to claim 9.

Figure 1

1A    PVP/KP188
      Dex/Gent

1B

Figure 1 (continued)

1C

1D

Figure 2

Figure 3

Figure 4

4A

4B

Figure 5

5A

5B

Europäisches
Patentamt

European
Patent Office

Office européen
des brevets

# EUROPEAN SEARCH REPORT

**Application Number**

EP 21 18 1716

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/269391 A1 (BANGO JR JOSEPH J [US] ET AL) 29 October 2009 (2009-10-29) | 1-6,9-12 | INV.<br>A61K9/00 |
| Y | * paragraph [0045] - paragraph [0046] *<br>* claims 1,2,5,6,16 * | 1-7,9-12 | |
| X | US 2013/280307 A1 (FULLANA MATTHEW J [US] ET AL) 24 October 2013 (2013-10-24) | 1-6,9-12 | |
| Y | * paragraph [0068] *<br>* paragraph [0075] * | 1-7,9-12 | |
| X | WO 2017/085264 A1 (DERMTREAT APS [DK]) 26 May 2017 (2017-05-26) | 1-6,9-12 | |
| Y | * examples 1,6 *<br>* page 21, line 7 * | 1-7,9-12 | |

**TECHNICAL FIELDS SEARCHED (IPC)**

A61K

The present search report has been drawn up for all claims

| Place of search | Date of completion of the search | Examiner |
|---|---|---|
| Munich | 2 December 2021 | Schüle, Stefanie |

CATEGORY OF CITED DOCUMENTS

X : particularly relevant if taken alone
Y : particularly relevant if combined with another
　document of the same category
A : technological background
O : non-written disclosure
P : intermediate document

T : theory or principle underlying the invention
E : earlier patent document, but published on, or
　after the filing date
D : document cited in the application
L : document cited for other reasons

&　: member of the same patent family, corresponding
　document

EPO FORM 1503 03.82 (P04C01)

## ANNEX TO THE EUROPEAN SEARCH REPORT
## ON EUROPEAN PATENT APPLICATION NO.

EP 21 18 1716

This annex lists the patent family members relating to the patent documents cited in  the above-mentioned European search report.
The members are as contained in the European Patent Office EDP file on
The European Patent Office is in no way liable for these particulars which are merely given for the purpose of information.

02-12-2021

| Patent document cited in search report | | Publication date | Patent family member(s) | | Publication date |
|---|---|---|---|---|---|
| US 2009269391 | A1 | 29-10-2009 | NONE | | |
| US 2013280307 | A1 | 24-10-2013 | NONE | | |
| WO 2017085264 | A1 | 26-05-2017 | AU | 2016358266 A1 | 05-04-2018 |
| | | | AU | 2018220020 A1 | 06-09-2018 |
| | | | BR | 112018001978 A2 | 18-09-2018 |
| | | | CA | 2993377 A1 | 26-05-2017 |
| | | | CN | 108135833 A | 08-06-2018 |
| | | | EP | 3334412 A1 | 20-06-2018 |
| | | | HK | 1256209 A1 | 13-09-2019 |
| | | | JP | 6606279 B2 | 13-11-2019 |
| | | | JP | 6929916 B2 | 01-09-2021 |
| | | | JP | 2018528256 A | 27-09-2018 |
| | | | JP | 2020022787 A | 13-02-2020 |
| | | | KR | 20180040704 A | 20-04-2018 |
| | | | KR | 20190020173 A | 27-02-2019 |
| | | | KR | 20190067280 A | 14-06-2019 |
| | | | MY | 167777 A | 25-09-2018 |
| | | | NZ | 740615 A | 28-06-2019 |
| | | | RU | 2018109496 A | 19-09-2019 |
| | | | RU | 2019129082 A | 28-04-2020 |
| | | | SG | 10201913222X A | 30-03-2020 |
| | | | US | 2018221295 A1 | 09-08-2018 |
| | | | US | 2019254985 A1 | 22-08-2019 |
| | | | WO | 2017085264 A1 | 26-05-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019148291 A1

**Non-patent literature cited in the description**

- *Delivery of Drugs: A Review. Polymers (Basel),* 13 May 2020, vol. 12 (5), 1114
- **ALMEIDA H et al.** Applications of poloxamers in ophthalmic pharmaceutical formulations: an overview. *Expert Opin Drug Deliv.,* September 2013, vol. 10 (9), 1223-37
- **GÖTTEL et al.** Electrospun nanofibers - A promising solid in-situ gelling alternative for ocular drug delivery. *Eur. J. Pharm. Biopharm.,* 2020, vol. 146, 125-132, https://doi.org/10.1016/j.ejpb.2019.11.012
- **GRIMAUDO et al.** Crosslinked Hyaluronan Electrospun Nanofibers for Ferulic Acid Ocular Delivery. *Pharmaceutics,* 2020, vol. 12, https://doi.org/10.3390/pharmaceutics12030274
- **VIGANI B et al.** Recent Advances in the Development of In Situ Gelling Drug Delivery Systems for Non-Parenteral Administration Routes. *Pharmaceutics,* 10 September 2020, vol. 12 (9), 859
- **A.A. AZARI ; N.P. BARNEY.** Conjunctivitis: a systematic review of diagnosis and treatment. *JAMA,* 2013, vol. 310, 1721-1729, https://doi.org/10.1001/jama.2013.280318
- **A.F. SMITH ; C. WAYCASTER.** Estimate of the direct and indirect annual cost of bacterial conjunctivitis in the United States. *BMC Ophthalmol,* 2009, vol. 9, 13, https://doi.org/10.1186/1471-2415-9-13
- **D. ACHOURI ; K. ALHANOUT ; P. PICCERELLE ; V. ANDRIEU.** Recent advances in ocular drug delivery. *Drug Dev. Ind. Pharm.,* 2013, vol. 39, 1599-1617, https://doi.org/10.3109/03639045.2012.736515
- **K. NAYAK ; M. MISRA.** A review on recent drug delivery systems for posterior segment of eye. *Biomed. Pharmacother,* 2018, vol. 107, 1564-1582, https://doi.org/10.1016/j.biopha.2018.08.138
- **J.M. TIFFANY.** The normal tear film. *Dev. Ophthalmol.,* 2008, vol. 41, 1-20, https://doi.org/10.1159/000131066
- **P.-L. DESTRUEL ; N. ZENG ; F. BRIGNOLE-BAUDOUIN ; S. DOUAT ; J. SEGUIN ; E. OLIVIER ; M. DUTOT ; P. RAT ; S. DUFAÿ ; A. DUFAY-WOJCICKI.** In Situ Gelling Ophthalmic Drug Delivery System for the Optimization of Diagnostic and Preoperative Mydriasis: In Vitro Drug Release, Cytotoxicity and Mydriasis Pharmacodynamics. *Pharmaceutics,* 2020, vol. 12, https://doi.org/10.3390/pharmaceutics12040360
- **N. MITTAL ; G. KAUR.** In situ gelling ophthalmic drug delivery system: Formulation and evaluation. *J. Appl. Polym. Sci.,* 2014, vol. 131, https://doi.org/10.1002/app.39788
- **Y. QIAN ; F. WANG ; R. LI ; Q. ZHANG ; Q. XU.** Preparation and evaluation of in situ gelling ophthalmic drug delivery system for methazolamide. *Drug Dev. Ind. Pharm.,* 2010, vol. 36, 1340-1347, https://doi.org/10.3109/03639041003801893
- **W. KIRCHER.** *Proper administration of topical ophthalmics Augenarzneimittel korrekt angewandt*
- **P. SAARINEN-SAVOLAINEN ; T. JÄRVINEN ; K. ARAKI-SASAKI ; H. WATANABE ; A. URTTI.** Evaluation of cytotoxicity of various ophthalmic drugs, eye drop excipients and cyclodextrins in an immortalized human corneal epithelial cell line. *Pharmaceutical research,* 1998, vol. 15, 1275-1280, https://doi.org/10.1023/a:1011956327987
- **A. KUMARI ; P.K. SHARMA ; V.K. GARG ; G. GARG.** Ocular inserts - Advancement in therapy of eye diseases. *J. Adv. Pharm. Technol. Res.,* 2010, vol. 1, 291-296, https://doi.org/10.4103/0110-5558.72419
- **J. SCHINDELIN ; I. ARGANDA-CARRERAS ; E. FRISE ; V. KAYNIG ; M. LONGAIR ; T. PIETZSCH ; S. PREIBISCH ; C. RUEDEN ; S. SAALFELD ; B. SCHMID.** Fiji: an open-source platform for biological-image analysis. *Nat. Methods,* 2012, vol. 9, 676-682, https://doi.org/10.1038/nmeth.2019

- **M.R.C. MARQUES ; R. LOEBENBERG ; M. ALMU-KAINZI.** Simulated Biological Fluids with Possible Application in Dissolution Testing. *Dissolution Technol,* 2011, vol. 18, 15-28, https://doi.org/10.14227/dt180311p15
- **J.P. CRAIG ; I. SINGH ; A. TOMLINSON ; P.B. MORGAN ; N. EFRON.** The role of tear physiology in ocular surface temperature. *Eye (Lond),* 2000, vol. 14, 635-641, https://doi.org/10.1038/eye.2000.156
- **C. PURSLOW ; J.S. WOLFFSOHN.** Ocular surface temperature: a review. *Eye & contact lens,* 2005, vol. 31, 117-123, https://doi.org/10.1097/01.icl.0000141921.80061.17
- **P.-L. DESTRUEL ; N. ZENG ; J. SEGUIN ; S. DOUAT ; F. ROSA ; F. BRIGNOLE-BAUDOUIN ; S. DUFAÿ ; A. DUFAY-WOJCICKI ; M. MAURY ; N. MIGNET.** Novel in situ gelling ophthalmic drug delivery system based on gellan gum and hydroxyethylcellulose: Innovative rheological characterization, in vitro and in vivo evidence of a sustained precorneal retention time. *International Journal of Pharmaceutics,* 2020, vol. 574, 118734, https://doi.org/10.1016/j.ijpharm.2019.118734
- **J. GUBERNATOR ; Z. DRULIS-KAWA ; A. KOZUBEK.** *A simply and sensitive fluorometric method for determination of gentamicin in liposomal suspensions,* 2006
- **A. GREINER ; J.H. WENDORFF.** Electrospinning: a fascinating method for the preparation of ultrathin fibers. *Angew. Chem. Int. Ed Engl.,* 2007, vol. 46, 5670-5703, https://doi.org/10.1002/anie.200604646
- **H. ALMEIDA ; M.H. AMARAL ; P. LOBÃO ; J.M. SOUSA LOBO.** Applications of poloxamers in ophthalmic pharmaceutical formulations: an overview. *Expert Opin. Drug Deliv.,* 2013, vol. 10, 1223-1237, https://doi.org/10.1517/17425247.2013.796360
- **Y.C. KIM ; M.D. SHIN ; S.F. HACKETT ; H.T. HSUEH ; R. LIMA E SILVA ; A. DATE ; H. HAN ; B.-J. KIM ; A. XIAO ; Y. KIM.** Gelling hypotonic polymer solution for extended topical drug delivery to the eye. *Nat. Biomed. Eng.,* 2020, vol. 4, 1053-1062, https://doi.org/10.1038/s41551-020-00606-8
- **A.A. AL-KINANI ; G. ZIDAN ; N. ELSAID ; A. SEYFODDIN ; A.W.G. ALANI ; R.G. ALANY.** Ophthalmic gels: Past, present and future. *Advanced Drug Delivery Reviews,* 2018, vol. 126, 113-126, https://doi.org/10.1016/j.addr.2017.12.017
- **M.A. MIRANDA ; P.D. MARCATO ; I.P.S. CARVALHO ; L.B. SILVA ; D.L. RIBEIRO ; R. AMARAL ; K. SWIECH ; J.K. BASTOS ; J.A.R. PASCHOAL ; R.B. DOS REIS.** Assessing the cytotoxic potential of glycoalkaloidic extract in nanoparticles against bladder cancer cells. *J. Pharm. Pharmacol.,* 2019, vol. 71, 1520-1531, https://doi.org/10.1111/jphp.13145
- **M.A. DA COSTA ; R.C. SEICEIRA ; C.R. RODRIGUES ; C.R.D. HOFFMEISTER ; L.M. CABRAL ; H.V.A. ROCHA.** Efavirenz dissolution enhancement I: co-micronization. *Pharmaceutics,* 2012, vol. 5, 1-22, https://doi.org/10.3390/pharmaceutics5010001
- **D.-G. YU ; L.-M. ZHU ; C.J. BRANFORD-WHITE ; J.-H. YANG ; X. WANG ; Y. LI ; W. QIAN.** Solid dispersions in the form of electrospun core-sheath polymer fibers. *Int. J. Nanomedicine,* 2011, vol. 6, 3271-3280, https://doi.org/10.2147/IJN.S27468
- **M.S. AW ; S. SIMOVIC ; J. ADDAI-MENSAH ; D. LOSIC.** Silica microcapsules from diatoms as new carrier for delivery of therapeutics. *Nanomedicine (Lond),* 2011, vol. 6, 1159-1173, https://doi.org/10.2217/nnm.11.29
- **P.O. NNAMANI ; F.C. KENECHUKWU ; E.U. DIBUA ; C.C. OGBONNA ; U.L. MONEMEH ; A.A. ATTAMA.** Transdermal microgels of gentamicin. *Eur. J. Pharm. Biopharm.,* 2013, vol. 84, 345-354, https://doi.org/10.1016/j.ejpb.2012.11.015
- **F. SHIEHZADEH ; M. TAFAGHODI ; M.-L. DEHGHANI ; F. MASHHOORI ; B.S. FAZLY BAZZAZ ; M. IMENSHAHIDI.** Preparation and Characterization of a Dry Powder Inhaler Composed of PLGA Large Porous Particles Encapsulating Gentamicin Sulfate. *Adv. Pharm. Bull.,* 2019, vol. 9, 255-261, https://doi.org/10.15171/apb.2019.029
- **A. KARAVA ; M. LAZARIDOU ; S. NANAKI ; G. MICHAILIDOU ; E. CHRISTODOULOU ; M. KOSTOGLOU ; H. IATROU ; D.N. BIKIARIS.** Chitosan Derivatives with Mucoadhesive and Antimicrobial Properties for Simultaneous Nanoencapsulation and Extended Ocular Release Formulations of Dexamethasone and Chloramphenicol Drugs. *Pharmaceutics,* 2020, vol. 12, https://doi.org/10.3390/pharmaceutics12060594
- **J.D. FERRY.** Viscoelastic properties of polymers. John Wiley & Sons, 1980
- **J.R. ROBINSON ; G.M. MLYNEK.** Bioadhesive and phase-change polymers for ocular drug delivery. *Advanced Drug Delivery Reviews,* 1995, vol. 16, 45-50

- **T.S. BHAMRA ; B.J. TIGHE.** Mechanical properties of contact lenses: The contribution of measurement techniques and clinical feedback to 50 years of materials development. *Cont. Lens Anterior Eye,* 2017, vol. 40, 70-81, https://doi.org/10.1016/j.clae.2016.11.005
- **I. TRANOUDIS ; N. EFRON.** Tensile properties of soft contact lens materials. *Cont. Lens Anterior Eye,* 2004, vol. 27, 177-191, https://doi.org/10.1016/j.clae.2004.08.002

- **J.A. SCHAFER ; L.B. HOVDE ; J.C. ROTSCHAFER.** Consistent rates of kill of Staphylococcus aureus by gentamicin over a 6-fold clinical concentration range in an in vitro pharmacodynamic model (IVPDM). *J Antimicrob Chemother,* 2006, vol. 58, 108-111, https://doi.org/10.1093/jac/dkl216
- **V.H. TAM ; S. KABBARA ; G. VO ; A.N. SCHILLING ; E.A. COYLE.** Comparative pharmacodynamics of gentamicin against Staphylococcus aureus and Pseudomonas aeruginosa. *Antimicrob. Agents Chemother.,* 2006, vol. 50, 2626-2631, https://doi.org/10.1128/AAC.01165-05